# EUROPÄISCHE PATENTSCHRIFT

(11) **EP 0 947 200 B1**
(45) Veröffentlichungstag und Bekanntmachung des Hinweises auf die Patenterteilung: **11.03.2015**
(21) Anmeldenummer: 99102340.9
(22) Anmeldetag: 04.02.1994
(51) Int. Cl.: A61K 38/09, A61P 15/08

(54) **Cetrorelixacetat enthaltende Zusammensetzung zur Behandlung weiblicher Infertilität und zur Gonadenprotektion**
Compositions comprising Cetrorelix acetate for the treatment of female infertility and gonadal protection
Compositions comprennant l'acétate de cetrorelix pour le traitement de l'infertilité chez les femmes et la protection des gonades

(30) Priorität: 19.02.1993 DE 4305225
(43) Veröffentlichungstag der Anmeldung: 06.10.1999
(62) Teilanmeldung aus: 94101672.7
(73) Patentinhaber: Zentaris IVF GmbH, 60314 Frankfurt am Main (DE)
(72) Erfinder: Engel, Jürgen, Prof. Dr., 63755 Alzenau (DE); Sauerbier, Dieter, 33824 Werther (DE); Wichert, Burkhard, Dr., 33615 Bielefeld (DE); Reissmann, Thomas, Dr., 60437 Frankfurt (DE)
(74) Vertreter: Nauwald, Gunter

(56) Entgegenhaltungen:
- EP-A- 0 268 066
- EP-A- 0 299 402
- WO-A-91/19743
- DD-A- 141 996
- SZENDE B ET AL: "Suppression of meiosis of male germ cells by an antagonist of luteinizing hormone-releasing hormone." PROCEEDINGS OF THE NATIONAL ACADEMY OF SCIENCES OF THE UNITED STATES OF AMERICA. UNITED STATES FEB 1990, Bd. 87, Nr. 3, Februar 1990 (1990-02), Seiten 901-903, XP001156025 ISSN: 0027-8424
- SCHALLY A. V. ET AL: "Protective effects of analogs of luteinizing hormone-releasing hormone against x-radiation-induced testicular damage in rats" PROC. NATL. ACAD. SCI. USA, Bd. 84, Februar 1987 (1987-02), Seiten 851-855, XP001157014
- KARASHIMA T. ET AL: "Protective effects of analogs of luteinizing hormone-releasing hormone against chemotherapy-induced testicular damage in rats" PROC. NATL. ACAD. SCI. USA, Bd. 85, April 1988 (1988-04), Seiten 2329-2333, XP001157013
- KAMISHKE A. ET AL: "Gonadal protection from radiation by GnRH antagonist or recombinant human FSH: a controlled trial in a male nonhuman primate (Macaca fascicularis)" J. ENDOCRINOLOGY, Bd. 179, 2003, Seiten 183-194, XP008026059

## Beschreibung

Cetrorelix ist ein Dekapeptid mit einer endständigen Säureamidgruppe, das als Acetatsalz eingesetzt wird. Die Synthese und einige pharmakologische Wirkungen etwa die Fruchtbarkeitshemmung und/oder -kontrolle oder die reversible Unterdrückung der gonadalen Aktivität während Bestrahlungs- oder Chemotherapie sind in der EP-Anmeldung EP 0 299 402 A2 beschrieben.

Szende B. et al.; Proc. Natl. Acad. Sci. USA, Bd. 87, Nr. 3, Februar 1990 (1990-02), Seiten 901-903 untersucht die Hemmung der Reifeteilung männlicher Keimzellen. Dabei wird die Wirkung von Cetrorelixbase auf Nacktmäuse geprüft und die Anwendung der Hemmwirkung bei männlicher Kontrazeption und der Schutz von Keimzellen gegen schädigende cytotoxische Mittel und Strahlen erörtert.

Schally A. V. et al.; Proc. Natl. Acad. Sci. USA, Bd. 84, Februar 1987 (1987-02), Seiten 851-855 beschreibt die mögliche Schutzwirkung des Agonisten [D-Trp⁶]LHRH sowie des Antagonisten *N*-Ac-[D-Phe(*p*Cl)^{1,2},D-Trp³,D-Arg⁶,D-Ala¹⁰]LHRH gegenüber Keimzellenschädigungen durch Strahleneinwirkung an Ratten. Die Ergebnisse an Ratten legen nahe, dass eine Vorbehandlung mit dem genannten Antagonisten und möglicherweise auch dem Agonisten eine verminderte Schädigung der Keimzellen bei Strahleneinwirkung zur Folge haben kann.

Karashima T. et al.; Proc. Natl. Acad. Sci. USA, Bd. 85, April 1988 (1988-04), Seiten 2329-2333 erörtert die mögliche Schutzwirkung des Agonisten [D-Trp⁶]LHRH sowie des Antagonisten *N*-Ac-[D-Phe(*p*Cl)^{1,2},D-Trp³,D-Arg⁶,D-Ala¹⁰]LHRH gegenüber Keimzellenschädigungen durch Einwirkung cytotoxischer Mittel an Ratten. Die Ergebnisse an Ratten legen nahe, dass eine Vorbehandlung mit den zwei genannten LHRH-Analogen eine Schutzwirkung auf die Keimzellen bei Einwirkung cytotoxischer Mittel zur Folge haben kann.

WO 91/19743 A (Applied Research Systems) 26. Dezember 1991 bescheibt eine kombinierte Anwendung eines GnRH-Antagonisten und GnRH zur Induktion einer Ovulation, wobei GnRH-Antagonist und GnRH entweder zum gleichen Zeitpunkt oder sequentiell aufeinander folgend verabreicht werden. Die Verabreichung soll bei polycyctischem Ovar oder hyperandrogener Erkrankung Anwendung finden.

DD 141 996 A (Tschauschev Peter) 4. Juni 1980 beschreibt ein Verfahren zur Herstellung von lyophilisierten Präparationen des Decapeptides "LHRH", die eine erhöhte zeitliche Stabilität erreichen. Die Lyophilisate sollen insbesondere in der Veterinärmedizin zur Ovulationssynchronisation eingesetzt werden. Zur Herstellung werden dem Decapeptid "LHRH" eine Trägersubstanz sowie eine Puffersubstanz zur Einstellung eines pH-Bereiches von 3,5-6,5 zugesetzt und nach üblicher Sterilfiltration erfolgt die Lyophilisation.

Gemäß vorliegender Anmeldung soll der Wirkstoff subcutan in einer Dosis von 0,1 bis 20 mg appliziert werden können. Die wässrigen Lösungen des Dekapeptids sind instabil, eine Autoklavierung im Endbehälter ist nicht möglich. Das Dekapeptid neigt unter arzneibuchüblicher Sterilisation zur Zersetzung. Um eine injizierbare Lösung zu erhalten, war daher die Entwicklung eines Lyophilisats erforderlich. Allerdings ist die Menge des Wirkstoffes in der zu lyophilisierenden Lösung so gering, dass bei niedrigen Wirkstoffkonzentrationen nur ein lockerer Flaum an der Glaswand der Ampulle nach der Trocknung der hilfsstofffreien Lösung resultiert, der mit dem zur Sterilisierung benutzten Wasserdampfstrom aus dem Vial ausgetragen wird. Es ist also erforderlich, einen Gerüstbildner einzusetzen, der einen stabilen Kuchen bildet. Bei hohen Konzentrationen kann auf diesen Hilfsstoff verzichtet werden. Als Gerüstbildner kommen folgende Hilfsstoffe infrage: Hexite, insbesondere Mannit, Glucit, Sorbit, wie D-Sorbit, Dulcit, Allit, Altrit (zum Beispiel D- und L-Altrit), Idit (zum Beispiel D- und L-Idit), deren optisch aktive Formen (D- beziehungsweise L-Formen) sowie die entsprechenden Racemate. Insbesondere wird Mannit, wie D-Mannit, L-Mannit, DL-Mannit, Sorbit und/oder Dulcit verwendet, und zwar hiervon vorzugsweise D-Mannit. Als Hexit können auch Mischungen der genannten Hexite verwendet werden, zum Beispiel Mischungen von Mannit und Sorbit und/oder Dulcit. Da Dulcit weniger wasserlöslich ist als beispielsweise Mannit, soll der Dulcitgehalt in der wässrigen Lösung beispielsweise 3 Gewichtsprozent nicht überschreiten. Mannit und Sorbit hingegen können beispielsweise in allen Verhältnissen gemischt werden.

Neben dem Hexit können auch noch andere, übliche pharmazeutische Hilfssstoffe zugeführt werden, wie zum Beispiel Aminosäuren, wie beispielsweise Alanin, Glycin, Lysin, Phenylalanin, Asparaginsäure, Glutaminsäure, Leucin, Lactose, Polyvinylpyrrolidon, Glukose, Fructose, Albumin und äquivalente gerüstbildende Soffe. Weiter können Harnstoff und Natriumchlorid als Gerüstbildner verwendet werden. Die Gesamtmenge an solchen Stoffen in der Lösung, die für die Gefriertrocknung eingesetzt wird, ist beispielsweise 0- 16,9 Gewichtsteile, beispielsweise 0,1 - 7 Gewichtsteile, bezogen auf 1 Gewichtsteil Cetrorelix. In dem fertigen Lyophilisat kann die Gesamtmenge an solchen Hilfsstoffen bis zu 16,9 Gewichtsteile, bezogen auf einen Gewichtsteil Hexit, betragen. Im einzelnen richtet sich die Menge an solchen Hilfsstoffen nach der vorhandenen Menge Hexit und zwar derart, daß die Gesamtmenge an Hexit und solchen anderen Hilfsstoffen in dem fertigen Lyophilisat maximal nicht mehr als 17 Gewichtsteile beträgt, bezogen auf 1 Gewichtsteil Cetrorelix. Falls in dem Lyophilisat nur 0,1 Gewichtsteile Hexit vorliegen, können also bis zu 16,9 Gewichtsteile an anderen Hilfsstoffen vorliegen; falls beispielsweise 8,5 Gewichtsteile Hexit vorliegen, kann zum Beispiel die Menge an anderen Hilfsstoffen bis zu 8,5 Gewichtsteile, bezogen auf 1 Gewichtsteil Cetrorelix, betragen.

Während der Entwicklungsarbeiten zu dem Lyophilisat mußte jedoch festgestellt werden, daß sich der Wirkstoff bei der Verarbeitung sehr unterschiedlich und nicht vorhersehbar verhält. Die ersten Ansätze führten zu guten Resultaten, bald jedoch stellte sich heraus, daß bei der Sterilfiltration Schwierigkeiten auftreten und Fehlchargen resultierten.

Aus der Literatur beispielsweise aus Powell, M. F.: Pharmaceutical Research, 1258-1263 (8) 1991; Dathe, M.: Int. J. Peptide Protein Res. 344-349 (36) 1990; Szejtli, J.: Pharmaceutical Technology International 16-22, 1991 ist bekannt, daß Oligopeptide, besonders solche mit endständiger Säureamidfunktion, zur Gelbildung neigen. Bei der Sterilfiltration ist dies an der Filtrationsgeschwindigkeit erkennbar, oft erkennt man sogar schon organoleptisch die erhöhte Viskosität derartiger Lösungen. Auf dem Sterilfilter bleibt eine gallertartige Schicht zurück. Damit ist es nicht mehr möglich, ein Arzneimittel mit einem genau definierten Gehalt an Wirkstoff herzustellen.

In der Tabelle 1 sind verschiedene Ergebnisse der ersten 11 Ansätze aufgelistet.

Die Wirkstoffgehalte schwanken zwischen 100 % und 36 %.

**Tabelle 1: Cetrorelix-Acetat**

| Charge | Dosierung | Wirkstoffgehalt % |
|---|---|---|
| 1 | 100 µg | 100 |
| 2 | 500 µg | 100 |
| 3 | 500 µg | 90 |
| 4 | 500 µg | 36 |
| 5 | 500 µg | 100 |
| 6 | 500 µg | 85 |
| 7 | 1 mg | 80 |
| 8 | 1 mg | 100 |
| 9 | 2 mg | 100 |
| 10 | 2 mg | 80 |
| 11 | 2 mg | 100 |

Um diese Gelbbildung zu vermeiden, werden in der Literatur folgende Zusatzstoffe aufgeführt, die versuchsweise eingesetzt wurden:
Es kommen organische Lösungsmittel infrage, beispielsweise Acetonitril, n-Butanol, tertiäres Butanol, Ethanol, Isopropanol, Octanol und Benzylalkohol. Weiter können Salze und Pufferlösungen verwendet werden, wie zum Beispiel Acetatpuffer, Citratpuffer, Natriumchlorid, Natriumphosphat, Natrium EDTA, Natriumbicarbonat, Phosphatpuffer, Guanidinacetat, Harnstoff.

Weitere Anwendung können Polymere finden, wie zum Beispiel Gelatine, Polyethylenglykol 600, Hydroxyethylstärke, Polyvinylpyrrolidon, Polyvinylalkohol. Auch der Zusatz von Aminosäuren, beispielsweise Alanin, Glycin, Lysin, Phenylalanin, Asparaginsäure, Glutaminsäure und Leucin wurde schon beschrieben. An Säuren wurde eingesetzt Citronensäure, Caprylsäure, Octansäure, Salzsäure, Schwefelsäure und Essigsäure. An physiologisch unbedenklichen Tensiden stehen Benzalkoniumchlorid, Cetylalkohol, Gallensäuren, Lecithine, Polysorbate, Spans^{(R)} und Pluronics^{(R)} zur Verfügung.

Auch Kohlehydrate und Cyclodextrine wie zum Beispiel Glukose, Lactose, Mannitol, Saccharose, alpha-, beta- und gamma Cyclodextrine, Hydroxypropyl-alpha-und beta-Cyclodextrine, Hydroxyethyl Cyclodextrine und Methyl-Cyclodextrine wurden schon eingesetzt. Diese Hilfsstoffe wurden als Filtrationshilfsmittel zur Vermeidung der Gelbildung erprobt.

Eine befriedigende Lösung des Problems war jedoch nicht zu erkennen. Lediglich ein Ansäuern mit Essigsäure zeigte Teilerfolge. Aber auch hier mußten immer wieder höhere Filtrationsverluste hingenommen werden.

Überraschenderweise stellte sich nun heraus, daß man Cetrorelix in 30 %iger VolumenNolumen Essigsäure gut lösen kann. Diese Lösung wird anschließend auf eine Endkonzentration von 3 % Cetrorelix mit Wasser für Injektionszwecke aufgefüllt und Mannit hinzugegeben. Obwohl in der Literatur beschrieben wird, daß in saurem Medium die endständige Amidgruppe leicht hydrolysiert, konnte dies beim Cetrorelix nicht festgestellt werden. Lösungen, die nach dieser Methode hergestellt werden,

machten bei der Filtration keinerlei Schwierigkeiten. Es wurden immer die korrekten Wirkstoffgehalte gefunden.

Die Filtrationsgeschwindigkeit erreicht Werte, die befriedigende Produktionsabläufe sicherstellen. Ein allgemeiner Prozeß zur sterilen Lyophilisation wird in Sucker, Fuchs und Speiser (Herausgeber) "Pharmazeutische Technologie" 2. Auflage 1991, Thieme-Verlag, Stuttgart-New York, auf den Seiten 557-559 beschrieben. Eine weitere Beschreibung des angewandten Lyophilisierungsprozesses findet sich in der DE-OS 37 35 614.

Das Lyophilisat findet seine Anwendung in der Therapie der weiblichen Sterilität. Ein Therapieverfahren besteht bisher darin, durch humanes Menopausen-Gonadotropin die Follikelreifung zu stimulieren und danach durch Gabe von humanem Chorion Gonadotropin die Ovulation auszulösen. Die dadurch ausgelöste Ovulation erfolgte 32 Stunden später. Die dadurch gewonnenen Eizellen stehen für die extracorporale Befruchtung zur Verfügung.

Ein Nachteil dieser Therapie mit Agonisten ist die Tatsache, daß in der Stimulationsphase bis zu 10 Follikel heranreifen. Durch diese erhöhte Follikelreifung kommt es zu Hormonspiegelspitzen des LH. Diese Peaks haben ein Frühstadium der Follikelreifung zur Folge und eine Ovulation zum nicht vorhergesehenen Zeitpunkt. Diese Ovulationsstörung tritt in ca. 25 % der behandelten Fälle auf und stellt einen Nachteil dar, da der Zyklus, der eine solche Ovulationsstörung zeigt, für die Gewinnung von Eizellen verloren ist und für die ganze Behandlung ca. 1 Monat später wiederholt werden muß.

Ein weiterer Nachteil der konventionellen Stimulationsbehandlung ist die lange Behandlungsdauer von 4 Wochen, die notwendig ist, um die befriedigende Suppression zu erreichen. Die Agonisten zeigen weiterhin in 1-2 % der Fälle einen Hyperstimulationssyndrom, bei der die Follikelzellen hypertrophieren. Das Risiko der Hyperstimulation ist besonders groß bei polyzystischen Ovarien. Das Hyperstimulationssyndrom ist eine schwere Nebenwirkung, die zu Todesfällen führen kann.

Es hat sich nun herausgestellt, daß der Antagonist Cetrorelix gerade bei dieser Behandlung folgende Vorteile aufweist:

### Beispiel 1 Fertilisationsstörungen:

Um eine totale Suppression zu erreichen, genügen bei der Behandlung mit Cetrorelix eine Behandlungsdauer von 5 Tagen. Das Hyperstimulationssyndrom kann nicht auftreten. Darüber hinaus läßt sich in der 2. Therapiephase, der Ovulationsauslösephase, HMG einsparen. Dies stellt einen nicht unbeträchtlichen Kostenvorteil dieser in-vitro-Fertilisationsbehandlung dar. Die in-vitro-Fertilisation wird beispielsweise beim Vorliegen einer Tubenanomalie eingesetzt. Für diese Therapie ist es notwendig, den Zyklus genau zu kontrollieren und den Zeitpunkt der Ovulation möglichst genau festzulegen. Bisher wurde dies nur eingeschränkt erreicht, da durch Stimulation mit HMG/HCG der präovulatorische LH-Anstieg oft zu früh auftrat oder nicht lange genug aufrechterhalten wurde. Es ist jedoch von entscheidender Bedeutung für den Behandlungserfolg, diesen verfrühten Anstieg zu verhindern, um den Befruchtungszeitpunkt genau festlegen zu können. Dadurch wird die körperliche und psychische Belastung der Patientinnen reduziert und die Kliniklogistik optimal eingesetzt. Um dieses Ziel mit hoher Zuverlässigkeit zu erreichen, ist es notwendig, die endogene Hormonproduktion (LH-FSH, Estradiol) möglichst vollständig zu supprimieren, um gleichzeitig durch die Gabe exogener Gonadotropine (HMG/HCG) die Follikelreifung stimulieren und den Hormonstatus jederzeit zu kontrollieren. Erst bei Erreichen einer genügend hohen Anzahl von Follikeln (4-6), die etwa den gleichen Reifegrad besitzen, wird durch die Gabe einer HCG-Bolusinjektion die Ovulation ausgelöst.

Durch den Einsatz eines Antagonisten läßt sich die Behandlung wesentlich erfolgreicher und sicherer für die Patientinnen gestalten.

### Beispiel 2 Gonadenprotektion:

Ein weiteres Einsatzgebiet des Cetrorelix-Lyophilisats gemäß der vorliegenden Erfindung ist die Verwendung bei der Gonadenprotektion von männlichen Patienten. Die männlichen Patienten werden mit Cetrorelix-Lyophilisat vorbehandelt, und die Aktivität der Gonaden wird unterstützt. Dadurch haben andere schädigende Noxen, wie beispielsweise eine Strahlentherapie oder eine Cytostatika-Therapie keine oder nur noch geringe Möglichkeiten, auf das empfindliche Gonaden-Gewebe einzuwirken.

### Beispiel 3 Herstellverfahren:

In ein geeignetes Glasgefäß werden ca. 1,5 Liter Wasser für Injektionszwecke vorgelegt. In ein weiteres Glasgefäß werden 210 g Wasser für Injektionszwecke vorgelegt und 91,17 g Essigsäure zugefügt. Die berechnete Menge Cetrorelixacetat (1,62 - 1,695 g, je nach Gehalt der eingesetzten Charge) wird in der hergestellten 30 %igen Essigsäure unter Rühren gelöst. Die Lösung wird in das Glasgefäß mit 1,5 Liter Wasser für Injektionszwecke überführt, 82,2 g Mannitol zugegeben, gelöst und mit Wasser für Injektionszwecke auf 3039 g aufgefüllt.

### Inprozeßkontrollen:

| | |
|---|---|
| pH-Wert: | 2,5 - 3,0 |
| Dichte: | 1,009 - 1,017 g/cm³ bei 20 °C |
| Brechungsindex: | 1,227 - 1,340 bei 440 nm und 20 °C |

Die Sterilisationen der Lösung erfolgt durch Filtration über ein geeignetes Membranfilter (Porenweite 0,2 µm) unter aseptischen Bedingungen. 100 ml Vorlauf sind zu verwerfen. Die Filter sind mit gespannten Wasserdampf zu sterilisieren. Cetrorelix Gefriertrocknungslösung wird vor Rekontamination geschützt aufbewahrt. Die Lösung wird unverzüglich in Injektionsflaschen DIN 2R farblos, hydrolytische Klasse I unter aseptischen Bedingungen dosiert und mit sterilen Gefriertrocknungsstopfen versehen. Die Sollfüllmenge beträgt 2,0 ml = 2,026 g.

Die 2 ml Injektionsflaschen wurden auf einer Injektionsflaschenwaschmaschine gespült und mit Heißluft getrocknet und sterilisiert. Die gereinigten Gefriertrocknungsstopfen wurden autoklaviert. Die vorverschlossenen Injektionsflaschen wurden in eine Gefriertrocknungsanlage überführt und bei einer Plattentemperatur von -40 °C eingefroren. Die Trocknung erfolgt mittels einer Plattentemperatur von -40 °C auf +20 °C steigend. Anschließend wird die Anlage mit sterilem Stickstoff geflutet, die Flaschen in der Anlage verschlossen, und die Stopfen mit Bördelkappen gesichert.

Die Injektionsflaschen werden visuell auf Verschlußfehler und äußere Fehler kontrolliert. Fehlerhafte Injektionsflaschen werden aussortiert und vernichtet.

Cetrorelix-Lyophilisat 1 mg ist ein weißer, fester Gefriertrocknungskuchen in einer farblosen 2 ml Injektionsflasche, die mit grauen Gefriertrocknungsstopfen und gelber Flipp-off-Bördelkappe verschlossen ist.

## Patentansprüche

1. Steriles Cetrorelixacetat-Lyophilisat, erhältlich durch Lösen von Cetrorelixacetat in 30% (v/v) Essigsäure, Auffüllen mit Wasser für Injektionszwecke auf eine Endkonzentration von 3% Cetrorelix und Zugabe von Mannitol, Sterilisation der Lösung durch Filtration über einen Membranfilter und anschließende Lyophilisierung.

2. Verwendung eines sterilen Cetrorelixacetat-Lyophilisats gemäß Anspruch 1 zur Herstellung eines Mittels zur Behandlung von weibliche Infertilität.

3. Verwendung gemäß Anspruch 2, wobei während einer ovariellen Stimulationsbehandlung der Ovulationszeitpunkt durch die Cetrorelixgabe kontrolliert wird, indem ein präovulatorischer LH Anstieg vermieden wird und anschließend nach Reifung der Follikel die Ovulation durch Gabe exogener Gonadotropine ausgelöst wird.

4. Verwendung eines sterilen Cetrorelixacetat-Lyophilisats gemäß Anspruch 1 zur Herstellung eines Mittels zur Gonadenprotektion von männlichen Patienten gegen Keimzellen schädigende Noxen wie Strahlenbehandlung und Chemotherapie mit Cytostatika.

## Claims

1. Sterile cetrorelix acetate lyophilisate, obtainable by dissolving cetrorelix acetate in 30% (v/v) acetic acid, making up with water for injections to a final concentration of 3% cetrorelix and adding mannitol, sterilising the solution by filtration through a membrane filter and subsequent lyophilisation.

2. Use of a sterile cetrorelix acetate lyophilisate according to Claim 1 for preparing a composition for treating female infertility.

3. Use according to Claim 2, where, during an ovarial stimulation treatment, the ovulation time is controlled by the administration of cetrorelix by avoiding pre-ovulatory LH increase and then, after follicle maturation, triggering ovulation by administration of exogenic gonadotropines.

4. Use of a sterile cetrorelix acetate lyophilisate according to Claim 1 for preparing a composition for gonad protection of male patients against germ cell-damaging noxae such as radiation treatment and chemotherapy with cytostatics.

## Revendications

1. Lyophilisat stérile d'acétate de cétrorélix, pouvant être obtenu par dissolution d'acétate de cétrorélix dans de l'acide acétique à 30 % (v/v), complément avec de l'eau pour injection jusqu'à une concentration finale de 3 % de cétrorélix et addition de mannitol, stérilisation de la solution par filtration sur un filtre à membrane et lyophilisation subséquente.

2. Utilisation d'un lyophilisat stérile d'acétate de cétrorélix selon la revendication 1 pour la fabrication d'un agent destiné au traitement de l'infertilité féminine.

3. Utilisation selon la revendication 2, dans laquelle au cours d'un traitement de stimulation ovarienne le moment de l'ovulation est régulé par la prise de cétrorélix en empêchant une augmentation pro-ovulatoire de LH, et ensuite après maturation des follicules l'ovulation est déclenchée par prise de gonadotropines exogènes.

4. Utilisation d'un lyophilisat stérile d'acétate de cétrorélix selon la revendication 1 pour la fabrication d'un agent destiné à la protection des gonades de patients masculins contre des agents nocifs endommageant les cellules germinales, tels qu'une radiothérapie et une chimiothérapie avec des agents cytostatiques.
